# FASCICULE DE BREVET EUROPEEN

(11) **EP 0 711 841 B1**
(45) Date de publication et mention de la délivrance du brevet: **14.10.1998**
(21) Numéro de dépôt: 95402537.5
(22) Date de dépôt: 13.11.1995
(51) Int. Cl.: C12Q 1/68

(54) **Séquences nucléotidiques hybridant spécifiquement avec une séquence nucléique génomique de Campylobacter coli**
Nukleotidsequenzen die spezifisch mit einer genomischen Sequent von Campylobacter coli hybridisieren
Nucléotide sequences hybridising specifically with a genomic sequence of Campylobacter coli

(30) Priorité: 14.11.1994 FR 9413622
(43) Date de publication de la demande: 15.05.1996
(73) Titulaire: INSTITUT PASTEUR, 75724 Paris Cédex 15 (FR)
(72) Inventeur: Stonnet, Véronique, F-92600 Asnieres (FR); Guesdon, Jean-Luc, F-92310 Sevres (FR)
(74) Mandataire: Le Guen, Gérard

(56) Documents cités:
- EP-A- 0 232 085
- EP-A- 0 350 392
- JOURNAL OF CLINICAL MICROBIOLOGY, vol. 31, no. 12, WASHINGTON, US, pages 3340-3343, XP000563916 EYERS ET AL.: "Discrimination among thermophilic Campylobacter species by PCR of 23S rRNA gene fragments"
- JOURNAL OF CLINICAL MICROBIOLOGY, vol. 27, no. 2, WASHINGTON, US, pages 321-326, XP000563805 CHEVRIER ET AL.: "Identification and classification of Campylobacter strains by using nonradioactive DNA probes"

## Description

La présente invention se rapporte à une séquence nucléique spécifique de *Campylobacter coli,* ainsi qu'aux applications de cette séquence en tant que sonde nucléotidique spécifique pour la détection de séquences de *Campylobacter coli* ou de fragments de cette séquence comme amorces nucléotidiques pour l'amplification d'ADN ou d'ARN de *Campylobacter coli* dans un échantillon biologique.

Les infections à *Campylobacter* sont très répandues dans le monde entier, affectant aussi bien l'homme que les animaux sauvages ou domestiques.

Bien que découvertes au début du vingtième siècle, les bactéries appelées actuellement *Campylobacter* furent longtemps méconnues, car leurs particularités rendaient leur identification et leur culture difficiles. D'abord isolés chez les ovidés et les bovidés et appelés *Vibrio fetus* puis, plus tard, *Campylobacter fetus,* ce n'est qu'à partir de 1946 que les premiers cas de campylobactérioses humaines ont été décrits, mais ce n'est qu'à partir de 1972, lorsque des milieux sélectifs pour *Campylobacter* ont commencé à être mis au point, que l'importance des infections à *Campylobacter* a pu être prouvée et reconnue.

Depuis la désignation de l'espèce type *Campylobacter fetus,* une douzaine d'autres espèces et sous-espèces ont été découvertes, le nombre exact variant selon les auteurs et les méthodes taxonomiques, qui proposent souvent de nouveaux critères de classification. Parmi ces espèces, les plus rencontrées en pathologie humaine et/ou animale sont *Campylobacter jejuni, Campylobacter coli* et *Campylobacter fetus.*

Actuellement, *Campylobacter jejuni et Campylobacter coli* sont fréquemment responsables de diarrhée infectieuse chez l'homme.

Le "réseau de surveillance nationale des infections à *Campylobacter",* mis en place en France en 1986, publie chaque année un bilan faisant ressortir les principales données épidémiologiques et cliniques des cas rapportés.

Dans l'espèce humaine, le symptôme majeur de l'infection intestinale à *C. jejuni* ou à *C. coli* est la diarrhée qui, dans les cas les plus graves peut entraîner de fortes pertes hydriques, ce qui peut être particulièrement dangereux pour les enfants et les nourrissons, très sensibles à la déshydratation. Cependant, l'entérite à *Campylobacter* reste souvent sans complications et les diarrhées peuvent même cesser spontanément au bout d'une semaine. Toutefois, les coprocultures peuvent rester positives jusqu'à quelques semaines ou même mois, et, dans 5 à 10% des cas, des rechutes peuvent survenir. Un traitement et un suivi rigoureux s'imposent donc, surtout pour les sujets immunodéprimés ou ayant des maladies graves (SIDA, cirrhose du foie, cancer, leucémie, etc.), chez lesquels les *Campylobacter* peuvent se comporter comme des bactéries opportunistes.

D'autres conséquences des infections à *C. jejuni ou C. coli* ont également été décrites, bien que plus rares ou exceptionnelles : adénite mésentérique, cholécystite, infections urinaires, méningites, septicémies, érythème noueux ou syndrome de Guillain-Barré, etc.

Chez les animaux, les *Campylobacter* vivent habituellement en commensaux dans le tube digestif de nombreuses espèces : bovins, ovins, porcs, volailles, oiseaux sauvages, chiens et chats. Ces animaux, malades ou porteurs sains, constituent un large réservoir de germes, et donc un risque important de contamination. Dans le cas d'infections évidentes, chez les bovins et ovins, *C. jejuni* est connu, depuis la première description en 1931, comme étant la cause de la "dysenterie du bétail", qui peut avoir pour conséquence, outre l'effet sur le bétail, la transmission à l'homme à travers la dissémination des germes dans l'environnement des animaux (terre, eaux). Même pour des animaux asymptomatiques, "porteurs sains", la transmission à l'homme peut se faire : soit par contact direct avec ces animaux ou leurs déjections, soit par consommation d'aliments ou d'eaux souillés (viandes contaminées pendant leur préparation et mal cuites, lait non pasteurisé, eau polluée, etc.).

Dans une optique de prévention, il est important donc, tant chez l'homme que chez l'animal, de pouvoir identifier le pathogène *C. jejuni* ou C. coli le plus tôt possible, afin d'empêcher, par des mesures adéquates, toute contamination. Ceci est particulièrement le cas dans l'industrie agro-alimentaire, où des conditions de stérilité doivent être respectées. C'est également important en pathologie humaine, pour effectuer un suivi correct des malades traités à la suite d'une infection à *Campylobacter,* afin d'éviter toute nouvelle rechute.

Enfin, dans le cas d'infections déclarées, il est très important de bien identifier le germe responsable, et ceci rapidement après le déclenchement de la maladie, afin de pouvoir appliquer un traitement correct et efficace qui empêcherait l'évolution de l'infection, voire la propagation d'épidémies. Or, l'identification des *Campylobacter* et la détermination de l'espèce incriminée n'est pas aisée. En effet, leur isolement nécessite des milieux spéciaux et leur détection classique ne se fait actuellement qu'après un enrichissement par culture d'au moins 48 heures. Ceci est très long lorsqu'un diagnostic rapide est nécessaire. D'autre part, étant donné que le diagnostic microbiologique se fait actuellement par des techniques bactériologiques et/ou biochimiques qui exploitent des différences phénotypiques existant entre les différentes espèces, des erreurs de diagnostic peuvent se produire, surtout lorsque des mutants apparaissent pour un caractère donné. Dans le cas précis de *C. jejuni* et *C. coli,* l'unique critère de différenciation est l'hydrolyse de l'hippurate (*C. jejuni* peut l'hydrolyser alors que *C. coli* ne le peut pas), et il arrive parfois que cette distinction ne puisse pas se faire, car il existe des souches *C. jejuni* hippurate-négatives (Hébert et al., J. Clin. Microbiol., 1984, 20, 138-140, Totten et al., J. Clin. Microbiol., 1987, 25, 1747-1752).

Des approches utilisant l'hybridation moléculaire pour identifier les espèces appartenant au genre *Campylobacter* ont été proposées. Cependant, ces méthodes ne permettent l'identification qu'après culture, elles ne sont pas suffisamment sensibles pour détecter cette bactérie dans les prélèvements biologiques. Ainsi, des méthodes d'identification et de classification des *Campylobacter* ont été proposées, utilisant soit des sondes radioactives (Ng et al., Mol. Cell. Probes, 1987, 1, 233-243), soit des sondes non radioactives (Chevrier et al., J. Clin. Microbiol., 1989, 27, 321-326), mais ces méthodes utilisent des sondes génomiques totales et nécessitent un enrichissement par culture du pathogène à détecter, car le seuil de détection est assez élevé, environ 10⁵ bactéries (Chevrier et al., ci-dessus).

Des recherches de sondes nucléiques spécifiques de *C. jejuni,* dans un but de diagnostic d'espèce, ont été faites par Picken et al. (Mol. Cell. Probes, 1987, 1, 245-259), Korolik et al. (J. Gen. Microbiol., 1988, 134, 521-529) et Zhou et Wang (Zbl. Bakt., 1989, 272, 186-190), mais il subsiste des problèmes de spécificité et les séquences de ces sondes potentielles n'ont pas été déterminées.

Récemment les inventeurs, auteurs de la présente invention ont découvert une séquence d'ADN spécifique de l'espèce *C. jejeuni* (FR-2 701 028). Cette séquence a été utilisée pour mettre au point un test PCR capable de détecter et d'identifier cette espèce dans un échantillon biologique (Stonnet et Guesdon, FEMS Immunol. Med. Microbiol., 1993, 7, 337-344). Compte tenu de la haute spécificité de la séquence, ce test PCR ne permet pas de détecter l'espèce *C. coli,* tout aussi importante cliniquement que *C. jejuni.* Récemment, une méthode d'identification et de discrimination spécifique parmi les *Campylobacter* thermophiles, dont *C. coli,* a été décrite par Eyers et al. (J. Clin. Microbiol., 1993, 31, 3340-3343). Toutefois, les amorces spécifiques d'espèce ont été choisies dans des régions hypervariables des gènes codant pour l'ARN ribosomique 23S, ce qui n'exclut pas une possibilité d'obtenir des résultats "faux-négatifs" lorsque l'ADN d'une souche à tester possède des variations juste au niveau de la séquence des amorces.

Toutefois ces raisons ont amené les inventeurs à choisir pour objectif d'isoler des séquences d'ADN spécifiques de *Campylobacter coli* et de développer à partir de ces séquences une méthode d'identification basée sur la technique PCR et la technique d'hybridation moléculaire. L'association de ces deux techniques permet d'atteindre un seuil de détection très bas, permettant de détecter et d'identifier une seule bactérie *C. coli* dans un échantillon biologique.

Les inventeurs ont à présent isolé une séquence nucléique utilisable pour la détection spécifique de l'espèce *Campylobacter coli.*

La présente invention a ainsi pour objet une séquence nucléotidique qui hybride spécifiquement avec l'acide nucléique génomique des souches appartenant à l'espèce *C. coli* et qui n'hybride pas dans les conditions habituelles avec les acides nucléiques de campylobactéries n'appartenant pas à cette espèce, ni avec les acides nucléiques génomiques de bactéries proches du genre *Campylobacter.*

L'invention a également pour objet une séquence d'acide nucléique ayant la séquence SEQ ID n° 1, la séquence complémentaire de celle-ci, ou une séquence qui en diffère par mutation, insertion, délétion ou substitution d'une ou plusieurs bases.

Par "séquence qui en diffère par mutation, insertion, délétion ou substitution d'une ou plusieurs bases", on entend une séquence qui hybride avec la séquence SEQ ID n° 1 ou sa séquence complémentaire dans les conditions de stringence habituelles définies par SAMBROOK J., FRITSCH E.F. et MANIATIS T. (1989) : Molecular Cloning : A Laboratory Manual, Ed. Cold. Spring Harbor Laboratory 9.47-9.62).

Ces conditions sont déterminées à partir de la température de stringence moyenne Tm.

De préférence, les séquences les plus avantageuses sont celles qui hybrident dans l'intervalle de température (Tm - 15°C) à (Tm - 20°C).

Les séquences en question comportent avantageusement au moins 12 nucléotides.

L'invention a également pour objet un vecteur de clonage contenant une séquence nucléotidique telle que définie ci-dessus, plus particulièrement le plasmide pCS1 contenant la séquence SEQ ID n° 1 déposé à la Collection Nationale de Culture de Microorganismes le 14 novembre 1994 sous le n° I-1491.

Les séquences nucléotidiques définies ci-dessus peuvent être des séquences d'ADN ou des séquences d'ARN.

La taille exacte du fragment de séquence SEQ ID n° 1 est de 604 pb.

Dans un premier temps, le fragment CS1 a été utilisé dans un test d'hybridation, suivant la technique de Southern, sur des ADNs génomiques de différentes souches de l'espèce *C. coli* (*C. coli* CIP 70.54, *C. coli* CIP 70.77, *C. coli* CIP 70.78, *C. coli* CIP 70.79, *C. coli* CIP 70.80, *C. coli* CIP 70.81, *C. coli* CIP 71,5, *C. coli* CIP 103753, *C. coli* A630-isolat clinique) digérés par l'enzyme *Hind*III, *Bgl*II, et *EcoR*V respectivement. Toutes les souches possèdent des fragments d'ADN qui hybrident avec la sonde CS1. Selon l'enzyme utilisée, *Hind*III*, Bgl*II*,* et *EcoR*V*,* ces fragments ont une taille de 2,3 kb, 3,5 kb ou 2,7 et 6 kb respectivement.

Dans un autre test de même type, le fragment CS1 a été utilisé comme sonde sur des ADNs cibles provenant d'autres espèces de *Campylobacter (Campylobacter jejuni, C. lari, C. fetus subsp. fetus, C. fetus subsp. venerealis, C. hyointestinalis, C. sputorum subsp. sputorum , C. sputorum subsp. bubulus, C. concisus, C. curvus* et *C. fecalis)* ou d'autres genres bactériens *(Escherichia coli, Arcobacter cryaerophylus, Helicobacter pylori, Salmonella typhimurium).* Ces ADNs cibles ont été digérés par l'enzyme *Hind*III*.* Une importante hybridation avec l'ADN de *C. coli* (témoin positif) a été observée, dès 3 h d'autoradiographie. Après 24 heures d'exposition, un léger signal est visible provenant de l'ADN de *C. jejuni.* Cette légère hybridation croisée n'est pas inexplicable, car il existe 30 à 50% d'homologie au niveau génomique entre *C. jejuni* et *C. coli .* Le fragment CS1 ne s'est hybridé à aucun des autres ADNs testés. Aucun signal n'était visible sur l'autoradiographie, même après des temps d'exposition très longs (72 heures).

La séquence SEQ ID n° 1 des parties ou des variants fonctionnellement équivalentes de celles-ci, peuvent être utilisés dans des techniques d'hybridation moléculaire pour la détection et l'identification de *Campylobacter coli.*

Les variants fonctionnellement équivalents comprennent des séquences dans lesquelles des paires de bases ont été mutées, délétées, insérées ou substituées, sans que les propriétés essentielles à la spécificité de ces fragments soient affectées.

Les séquences nucléotidiques selon l'invention ont des applications diagnostiques et épidémiologiques en médecine humaine ou vétérinaire, notamment comme sondes nucléiques spécifiques de *Campylobacter coli* ou comme amorces oligonucléotidiques pour l'amplification d'une séquence spécifique de *Campylobacter coli.*

Les sondes selon l'invention comprennent avantageusement au moins 20 nucléotides consécutifs parmi les séquences ou les fragments de séquences mentionnés ci-dessus.

Les sondes sont des sondes d'ADN ou des sondes d'ARN.

Les séquences nucléotidiques décrites dans cette invention peuvent ainsi être utilisées comme sondes pour détecter spécifiquement et de manière directe des souches de *Campylobacter coli* dans un échantillon biologique. Les séquences non marquées peuvent être utilisées directement comme sondes, cependant les séquences sont généralement marquées par un élément radioactif (³²p, ³⁵S, ³H, ¹²⁵I) ou par une molécule non-radioactive (biotine, acétylaminofluorène, digoxigénine, 5-bromo-désoxyuridine) pour obtenir des sondes utilisables pour de nombreuses applications.

Dans ce dernier cas, on pourra utiliser l'une des méthodes de marquage décrites dans FR 2 422 956 et FR 2 518 755. La technique d'hybridation peut être réalisée de manières diverses (Matthews, J.A. et Kricka, L.J., Anal. Biochem. 1988, 169, 1-25). La méthode la plus générale consiste à immobiliser l'acide nucléique extrait des cellules de *Campylobacter coli* sur un support (nitrocellulose, nylon, polystyrène,... ) et à incuber, dans des conditions bien définies, l'acide nucléique cible immobilisé avec la sonde. Après l'hybridation, l'excès de sonde est éliminé et les molécules hybrides formées sont détectées par la méthode appropriée (mesure de la radioactivité, de la fluorescence ou de l'activité enzymatique liée à la sonde...).

Dans une autre application, les sondes d'acide nucléique décrites ici peuvent être utilisées comme sondes de capture. Dans ce procédé, la sonde est immobilisée sur un support et sert à capturer par hybridation spécifique l'acide nucléique cible extrait de *C. coli.* Si nécessaire, le support solide est séparé de l'échantillon et le duplex formé entre la sonde de capture et l'acide nucléique cible est ensuite détecté grâce à une seconde sonde de détection marquée par un élément facilement détectable.

Lorsqu'une quantité suffisante d'acide nucléique de *Campylobacter coli* peut être extraite à partir des prélèvement à analyser, les séquences décrites dans le brevet sont utilisables pour détecter et identifier les souches appartenant à *Campylobacter coli* directement dans ces prélèvements. Dans le cas contraire, une culture rapide en milieu liquide peut être réalisée avant l'extraction de l'acide nucléique de *Campylobacter coli,* ou bien la petite quantité d'acide nucléique de *Campylobacter coli* extrait du prélèvement peut être soumise à une technique d'amplification comme par exemple la technique PCR.

La séquence SEQ ID n° 1 et ses séquences dérivées peuvent également être utilisées pour sélectionner des amorces oligonucléotidiques, notamment pour la technique PCR.

Cette technique nécessite le choix de paires d'oligonucléotides encadrant le fragment qui doit être amplifié (brevet U.S. n° 4 683 202). Ces amorces oligodé-soxyribonucléotidiques ou oligoribonucléotidiques ont avantageusement une longueur comprise entre 18 et 30 et de préférence 18 à 22 nucléotides. L'une des deux amorces est complémentaire du brin (+) de la matrice et l'autre amorce est complémentaire du brin (-). Il est important que ces amorces ne possèdent pas de structure secondaire ou de séquence complémentaire l'une de l'autre. D'autre part, la longueur et la séquence de chaque amorce doivent être choisies de manière à ce que les amorces ne s'hybrident pas avec d'autres acides nucléiques provenant de cellules procaryotes ou eucaryotes, en particulier avec les acides nucléiques des *Campylobacter* n'appartenant pas à l'espèce *coli* et avec l'ADN ou l'ARN humain pouvant éventuellement contaminer le prélèvement.

Les amplimères sélectionnés comme amorces spécifiques pour l'amplification de séquences nucléiques de souches appartenant à *Campylobacter coli* sont choisis par exemple en suivant la méthode décrite par Griffais et Coll. (Nucleic Acids Res. 1991, 19, 3887-3891).

A partir de la séquence SEQ ID n° 1, les inventeurs ont choisi des oligonucléotides pour réaliser un test PCR. Grâce à ces oligonucléotides, ils ont obtenu une amplification spécifique de *Campylobacter coli,* aucune amplification n'était visible avec l'ADN des 10 autres *Campylobacter* mentionnés ci-dessus, en particulier *C. jejuni,* ni avec l'ADN *d'Escherichia coli, Helicobacter pylori, Salmonella typhimurium, Arcobacter cryaerophilus.*

Un couple d'amorces tout particulièrement préféré est représenté par les oligonucléotides CSF et CSR issus de la séquence SEQ ID n° 1, de séquences :

Les fragments d'acide nucléique obtenus par amplification génique à l'aide des amorces décrites ci-dessus constituent également un objet de l'invention.

Les fragments amplifiés peuvent être identifiés après une électrophorèse en gel d'agarose ou de polyacrylamide, ou après une électrophorèse capillaire, ou encore après une technique chromatographique (filtration sur gel, chromatographie hydrophobe ou sur échangeur d'ions). La spécificité de l'amplification peut être contrôlée par hybridation moléculaire en utilisant comme sonde la séquence nucléotidique SEQ ID n° 1, des fragments de celles-ci, des plasmides contenant ces séquences ou des fragments de celles-ci, des oligonucléotides complémentaires de ces séquences ou fragments de séquences ou des produits d'amplification. Ces sondes peuvent être marquées ou non par des éléments radioactifs ou par des molécules non radioactives.

La présente invention a également pour objet une méthode de détection de la présence des souches de *Campylobacter coli* dans un échantillon biologique, caractérisée par les étapes suivantes :
i) mise en contact de l'échantillon biologique avec un couple de fragments oligonucléotidiques dits amorces, tels que définis précédemment, l'acide nucléique contenu dans l'échantillon ayant, le cas échéant, été préalablement rendu accessible à l'hybridation et dans des conditions permettant une hybridation des amorces à l'acide nucléique des souches appartenant à *Campylobacter coli* ;
ii) amplification de l'acide nucléique des souches de *Campylobacter coli* ;
iii) mise en évidence de l'amplification de fragments d'acide nucléique correspondant au fragment encadré par les amorces ;
iv) vérification éventuelle de la séquence du fragment amplifié, par exemple par hybridation de sonde spécifique, par séquençage ou par analyse de site de restriction.

La présente invention a en outre pour objet un kit ou coffret pour la détection de la présence de souches appartenant à *Campylobacter coli* dans un échantillon biologique, caractérisé en ce qu'il comporte les éléments suivants :
- un couple de fragments oligonucléotidiques tels que définis précédemment ;
- les réactifs nécessaires pour effectuer une amplification d'acide nucléique de souches appartenant à *Campylobacter coli ;*
- éventuellement, un composant permettant de vérifier la séquence du fragment amplifié, plus particulièrement une sonde nucléique telle que définie précédemment.

Ce kit contient plus avantageusement la ou les sondes marquées ou non. Celles-ci peuvent être en solution ou immobilisées sur un support. Le kit peut également contenir les réactifs nécessaires pour la lyse des bactéries et l'extraction des acides nucléiques cibles, ainsi que les solutions d'hybridation et de lavage correspondant à la méthode choisie.

L'invention est illustrée plus en détail dans les exemples qui suivent et les figures annexées sur les lesquelles :
- la fig. 1 représente les profils d'hybridation des ADNs de différentes souches de *C. coli* avec le fragment CS1 utilisé comme sonde ;
- la fig. 2 représente les résultats d'électrophorèse obtenus après amplification par PCR avec le couple d'amorces CSF et CSR.

### EXEMPLE 1 :

**Construction de la banque génomique de *C. coli.* Criblage de la banque et détermination de la séquence du fragment spécifique :**

L'ADN génomique de *C. coli* CIP 70.80 est digéré partiellement par l'endonucléase de restriction *Hind* III en faisant agir 0,5U d'enzyme par µg d'ADN dans le tampon recommandé par le fournisseur pendant 1 heure à 37°C. Ces conditions permettent l'obtention préférentielle de fragments de longueur comprise entre 30 et 40 kb. Après digestion, le mélange contenant les fragments d'ADN est purifié par extractions au phénol/chloroforme et l'ADN est précipité à l'éthanol.

Le vecteur est le cosmide pHC79. Il est digéré par l'enzyme *Hind* III et déphosphorylé pour éviter toute autoligation.

La ligation s'effectue en mélangeant 700 ng de vecteur et 3 µg de fragments d'ADN de 30/40 kb, le mélange est laissé à 12°C pendant 18 heures après que l'on ait ajouté 5 unités de T4 DNA ligase dans un tampon approprié.

Les cosmides recombinants sont encapsidés in vitro et utilisés pour transformer les bactéries (*E*. *coli* HB 101). Les bactéries transformées sont incubées 1 heure à 37°C en milieu LB, puis étalées sur milieu sélectif Agar contenant 25 µg/ml d'ampillicine. Les colonies résistantes à l'ampicilline sont toutes testées pour leur sensibilité à la tétracycline, en effet le fragment d'ADN de 30/40 kb est inséré dans le vecteur de manière à inactiver le gène de résistance à la tétracycline (Tet) et à conserver le gène de résistance à l'ampicilline (Amp).

Il est effectué une mini préparation d'ADN des 200 premières colonies transformantes résistantes à l'ampicilline (Amp^{r}) et sensibles à la tétracycline (Tet^{s}) selon la technique de la lyse alcaline. L'ADN de ces préparations est ensuite digéré par l'endonucléase de restriction *Hind* III, analysé en électrophorèse sur gel d'agarose à 0,8%, puis transféré sur des filtres de Nylon. L'ADN est fixé de façon irréversible par 3 minutes d'exposition aux UV à 254 nm.

Ces différents filtres sont incubés pendant 16-18 heures à 65°C dans un tampon 6X SSC (1X SSC correspond à 0,15M NaCl et 0,015M citrate de Na) contenant 10% de sulfate de dextrane, une solution de Denhardt 5X concentrée (une solution de Denhardt 1X correspond à 0,02% de Ficoll, 0,02% de polyvinylpyrrolidone et 0,02% d'albumine sérique bovine), 10mM EDTA, 0,5% de SDS, 100 µg/ml d'ADN dénaturé de sperme de saumon et l'ADN génomique, radiomarqué au ³²P par multipriming, de l'une des deux espèces suivantes : *C. jejuni* CIP 70.2, *C. coli* CIP 70.80.

Après hybridation, les filtres sont lavés 2 fois 10 minutes en 2X SSC à 65°C, 1 fois pendant 30 minutes en 2X SSC + 0,1% SDS à 65°C, et enfin 1 fois pendant 10 minutes en 0,1X SSC à 65°C. Les filtres encore humides sont mis en autoradiographie à -80°C avec écran intensifiant de 15 minutes à 3 jours.

Les résultats de ces hybridations ont permis d'isoler un clone cosmidique contenant un fragment d'environ 2,3 kb nommé CS1.

La spécificité du fragment a été vérifiée comme décrit dans l'exemple n° 2.

Le fragment CS1 a été séquencé selon la méthode de Sanger en utilisant le kit de séquençage "T7 Sequencing kit" (Pharmacia) directement sur le cosmide, après dénaturation alcaline des deux brins d'ADN. Toutes les réactions de séquençage ont été réalisées avec du dATP marqué au ³⁵S.

La partie séquencée (600 paires de bases) du fragment est représentée dans le listage joint à la description. La comparaison entre les banques de données "Genebank" et "EMBL" et les 600 nucléotides du fragment CS1 ainsi déterminés, ne fait ressortir aucune homologie significative avec les séquences connues aujourd'hui.

### EXEMPLE 2 :

**Analyse d'ADN par la technique de Southern, en utilisant comme sondes les séquences d'acide nucléique de l'invention :**

La liste et les références des bactéries utilisées dans cette étude sont les suivantes :

### Campylobacter:

*C. jejuni* subsp. *jejuni* CIP 70.2, CIP 70.86, CIP 81.1
*C. jejuni* subsp. *doylei* CIP 103751
*C. coli* CIP 70.80, CIP 71.5, CIP 70.81, CIP 70.79, CIP 70.78, CIPI 70.77, CIP 70.54
*C. lari* CIP 102722
*C. upsaliensis* CIP 103681
*C. fetus* subsp. *fetus* CIP 5395
*C. fetus* subsp. *venerealis* CIP 6829
*C. hyointestinalis* (isolat clinique)
*C. sputorum* subsp. *sputorum* CCUG 9728
*C. sputorum* subsp. *bubulus* CIP 53103
*C. concisus* (isolat clinique)
*C. fecalis* CIP 12014
*C. Curvus* (isolat clinique)

### Non-Campylobacter:

*Arcobacter cryaerophila* CCUG 17801
*Helicobacter pylori* CIP 101260
*Escherichia coli* HB101
*Salmonella* subsp. *typhimurium* C 53
*Salmonella* subsp. *salamae*
*Salmonella* subsp. *diarizonae*
*Enterococcus fecalis* JH2-SM
*Enterococcus faecium* D372 (isolat clinique)
*Bacteroides fragilis* E134 (isolat clinique)
*Bacteroides fragilis* F238 (isolat clinique)

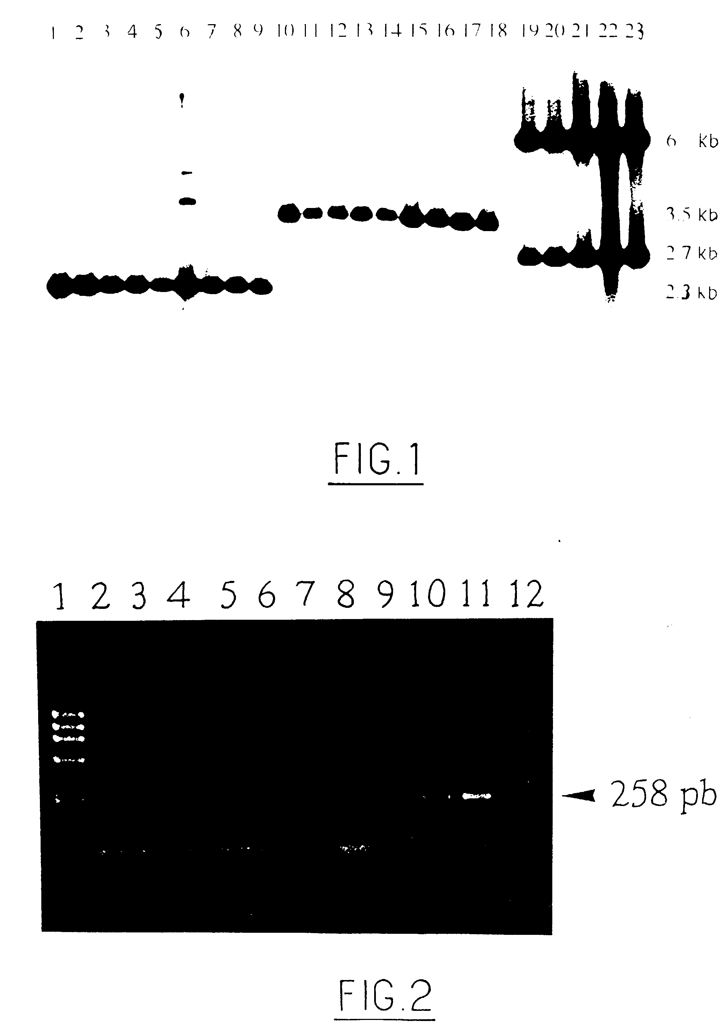

Les résulats sont représentés à la fig. 1, le numéro des pistes correspondant dans l'ordre aux souches ci-dessus énumérées.

### ADN de bactéries appartenant au genre Campylobacter, autres que C. coli:

Après culture en milieu approprié (gélose au sang de mouton à 5%, Biomérieux), les *Campylobacter* sont traités de la manière suivante : les bactéries de chaque boîte de Petri sont récoltées avec 2 ml de tampon TE-glucose (Tris-HCl pH 8 25 mM, EDTA 10 mM, glucose 50 mM), centrifugées pendant 5 minutes à 5000 g, le culot est redispersé et lavé en TE-glucose puis recentrifugé, les bactéries sont resuspendues dans 100 µl de tampon TE (Tris-HCl 10 mM pH8, EDTA 1 mM) et l'ADN est extrait selon la technique de Pitcher et al. (Lett. Appl. Micro-biol., 1989, 8, 151-156). Deux µg des ADN ainsi extraits sont soumis à une digestion totale par l'enzyme *Hind* III. Les fragments obtenus sont ensuite séparés par électrophorèse sur gel d'agarose à 0,8% en TAE avant d'être transférés sur membrane de Nylon selon la technique de Southern.

Les fragments transférés sont analysés par hybridation moléculaire. La sonde CS1, marquée par du ³²P, détecte spécifiquement l'ADN de *C. coli* et n'hybride pas avec les ADN génomiques d'autres *Campylobacter,* sauf très légèrement avec un fragment d'ADN situé sur le génome de *C. jejuni.* Cette hybridation croisée n'est détectable qu'après 18 heures d'exposition, alors que l'ADN de *C. coli* est détectable dès 3 heures d'exposition.

### ADN de bactéries n'appartenant pas au genre Campylobacter :

L'ADN de ces bactéries et de C. *coli* utilisé comme contrôle positif a été hydrolysé par l'enzyme de restriction *HindIII,* puis les fragments ont été séparés par électrophorèse en gel d'agarose et transférés sur une membrane de nylon Hybond-N. Ces différents fragments d'ADN sont analysés par hybridation moléculaire, en utilisant comme sonde le fragment CS1 marqué au ³²P selon la technique du kit "Random primed DNA labelling" (Boehringer). L'autoradiographie montre que la seule espèce détectée est C. *coli.* Aucune hybridation n'est détectable sur les ADN des espèces non-campylobactériennes, même après 72 heures d'exposition.

### EXEMPLE 3 :

**Amplification enzymatique *in* vitro de l'ADN de C. *coli* avec les amorces définies à partir de la séquence d'acide nucléique faisant l'objet de l'invention.**

### Choix des amorces :

Il a été démontré que c'est essentiellement l'extrémité 3' des amorces oligonucléotidiques qui détermine la spécificité de la PCR. Il est donc important que cette région 3' soit parfaitement spécifique de la cible à amplifier.

Etant donné que le génome de *Campylobacter* présente un très faible pourcentage de guanine et cytosine (entre 28 et 38% de G+C), les inventeurs ont pensé que des amorces dont l'extrémité 3' était riche en G+C pouvaient présenter un important degré de spécificité.

A l'aide d'un programme informatique les inventeurs ont recherché, à l'intérieur de la séquence CS1, des zones riches en G+C qui sont présentes une seule fois sur CS1. C'est partir de ces régions que la séquence des amorces a été orientée et complétée de façon à obtenir une longueur d'une vingtaine de nucléotides.

### Synthèse des amorces oligonucléotidiques :

Les amorces dérivées de la séquence CS1, appelées CSF et CSR et ayant une longueur de 22 nucléotides, ont été synthétisées à l'aide d'une méthode des phosphoramidites sur un automate par le service de Chimie organique de l'Institut Pasteur. La concentration de chaque amorce est déterminée au spectrophotomètre.

### Amplification :

La technique d'amplification enzymatique *in vitro* (PCR) est réalisée selon le protocole décrit par Saiki et al. (Science, 1988, 239, 487-491) en utilisant 1 µM des oligonucléotides CSF et CSR et 30-100 ng d'ADN de différentes souches de *Campylobacter* avec 0,5 unité de Taq polymérase dans un tampon contenant 25 mM de KCl, 20mM de Tris-HCl pH 8,5, 1,5 mM de MgCl₂, 200 µM de désoxyribonucléotides triphosphates et 100 µg/ml d'albumine sérique bovine, le volume final de la réaction étant de 50 µl. Les paramètres des étapes de la PCR ont été choisis de la façon suivante : 5 minutes à 94°C, 1 minute à 60°C, 1 minute à 72°C, puis (1 minute à 94°C, 1 minute à 60°C, 1 minute à 72°C) 38 fois et un dernier cycle 1 minute à 94°C, 1 minute à 60°C, 5 minutes à 72°C. Quarante cycles sont ainsi réalisés en utilisant un appareil automatique. Après le dernier cycle, les échantillons sont maintenus à 4°C jusqu'à l'analyse.

### Analyse électrophorétique sur un gel d'agarose des échantillons amplifiés :

Dix µl des échantillons amplifiés sont déposés sur un gel d'agarose à 2% dans un tampon TBE (0,04 M Tris-borate, 0,001M EDTA) contenant 1 µg/ml de bromure d'éthidium. Les fragments amplifiés sont visualisés sous UV et les gels sont photographiés en utilisant un film Polaroid 667.

Les résultats obtenus avec des ADNs de différentes espèces de *Campylobacter* et les amorces CSF et CSR montrent que la longueur du fragment amplifié obtenu correspond à la longueur théorique attendue avec ce couple d'amorces et qui est de 258 paires de bases.

En complément de ces résultats, aucun fragment amplifié n'est visible lorsque l'ADN analysé est extrait des espèces ou genres suivants :
*C. jejuni* subsp. *jejuni, C. jejuni* subsp. *doylei, C. lari, C. upsaliensis, C. fetus* subsp. *fetus, C. fetus* subsp. *venerealis, C. hyointestinalis, C. cryaerophila, C. sputorum* subsp. *sputorum, C. sputorum* subsp. *bubulus, C. fecalis, C. concisus, C. curvus, Escherichia coli, Helicobacter pylori, Arcobacter cryaerophilus, Salmonella* subsp. *salamae, Salmonella* subsp. *diarizonae, Enterococcus fecalis, Enterococcus faecium, Bacteroides fragilis* ou de cellules humaines. Bien entendu, toutes les souches de *C. coli* testées ont donné des résultats positifs, qu'il s'agisse de souches de collection ou d'isolats de malades.

En conclusion, l'ensemble de ces résultats démontre que, à l'aide de la technique PCR et des amorces décrites dans cette invention, il est possible de détecter spécifiquement l'espèce *C. coli,* ce qui laisse penser que des isolats de malades et des échantillons biologiques infectés par *C. coli* pourront être identifiés, rendant ainsi la méthode utilisable dans le domaine de la bactériologie clinique, en médecine vétérinaire et dans l'industrie agro-alimentaire.

## Revendications

1. Séquence d'acide nucléique qui hybride spécifiquement avec un acide nucléique génomique des souches appartenant à l'espèce *Campylobacter coli* et qui n'hybride pas dans les conditions habituelles avec un acide nucléique de campylobactéries n'appartenant pas à cette espèce, ni avec un acide nucléique génomique de bactéries proches du genre *Campylobacter,* caractérisée en ce qu'elle possède la séquence nucléotidique SEQ ID N° 1, la séquence complémentaire de celle-ci, ou une séquence qui en diffère par mutation, insertion, délétion ou substitution d'une ou plusieurs bases.

2. Vecteur de clonage caractérisé en ce qu'il contient une séquence nucléotidique selon la revendication 1.

3. Plasmide déposé à la Collection Nationale de Culture de Microorganismes, le 14 novembre 1994, sous le n° I-1491.

4. Sonde nucléique spécifique des acides nucléiques de *Campylobacter coli,* caractérisée en ce qu'elle comprend au moins 20 nucléotides consécutifs choisis parmi les séquences nucléotidiques selon la revendication 1.

5. Sonde nucléique selon la revendication 4, caractérisée en ce qu'elle est immobilisée sur un support et utilisée comme sonde de capture.

6. Couples d'amorces oligonucléotidiques spécifiques pour l'amplification d'une séquence nucléique de *Campylobacter coli,* caractérisés en ce qu'ils comprennent des paires d'oligonucléotides ayant de 18 à 30 nucléotides, de préférence 18 à 22 nucléotides choisis parmi les séquences selon la revendication 1.

7. Couple d'amorces oligonucléotidiques spécifiques pour l'amplification d'une séquence nucléique de *Campylobacter coli* selon la revendication 6, caractérisé en ce qu'il est constitué par les paires oligonucléotidiques de séquences :

8. Fragment d'acide nucléique obtenu en amplifiant un acide nucléique de C. *coli* par une technique d'amplification génique, notamment par la technique PCR, au moyen d'un couple d'amorces oligonucléotidiques selon la revendication 6 ou 7.

9. Méthode de détection de la présence de *Campylobacter coli* dans un échantillon biologique, caractérisée par les étapes suivantes :
a) mise en contact de l'échantillon biologique avec un couple d'amorces selon la revendication 6 ou 7, l'acide nucléique de *Campylobacter coli* contenu dans l'échantillon biologique ayant, le cas échéant, été rendu préalablement accessible à une hybridation, et dans des conditions permettant une hybridation des amorces à l'acide nucléique appartenant à *Campylobacter coli ;*
b) amplification de l'acide nucléique appartenant à *Campylobacter coli ;*
c) mise en évidence de l'amplification de fragments d'acide nucléique de *Campylobacter coli* correspondant au fragment encadré par les amorces ;
d) vérification éventuelle de la séquence du fragment amplifié, par exemple par hybridation de sonde spécifique, par séquençage ou par analyse de site de restriction.

10. Kit de détection de la présence de *Campylobacter coli* dans un échantillon biologique, caractérisé en ce qu'il comprend les éléments suivants :
- un couple d'amorces selon la revendication 6 ou 7 ;
- les réactifs nécessaires pour effectuer une amplification d'un acide nucléique de *Campylobacter coli ;*
- éventuellement un composant permettant de vérifier la séquence du fragment amplifié, plus particulièrement une sonde nucléique selon la revendication 4 ou 5.

## Patentansprüche

1. Nucleinsäuresequenz, die spezifisch mit einer genomischen Nucleinsäure der Stämme, die der Spezies Campylobacter coli angehören, hybridisiert, und die unter üblichen Bedingungen weder mit einer Nucleinsäure von Campylobakterien, die dieser Spezies nicht angehören, noch mit einer genomischen Nucleinsäure von Bakterien, die der Gattung Campylobacter nahestehen, hybridisiert, dadurch gekennzeichnet, daß sie die Nucleotidsequenz SEQ ID Nr.1, die dazu komplementäre Sequenz oder eine Sequenz aufweist, die sich davon durch Mutation, Insertion, Deletion oder Substitution von einer oder mehreren Basen unterscheidet.

2. Klonierungsvektor, dadurch gekennzeichnet, daß er eine Nucleotidsequenz nach Anspruch 1 aufweist.

3. Plasmid, hinterlegt bei der Collection Nationale de Culture de Microorganismes am 14. November 1994 unter der Nr. 1-1491.

4. Nucleinsonde, spezifisch für Nucleinsäuren von Campylobacter coli, dadurch gekennzeichnet, daß sie mindestens 20 aufeinanderfolgende Nucleotide aufweist, ausgewählt aus den Nucleotidsequenzen nach Anspruch 1.

5. Nucleinsonde gemäß Anspruch 4, dadurch gekennzeichnet, daß sie an einem Träger immobilisiert ist und als Abfangsonde verwendet wird.

6. Oligonucleotidische Primerpaare, spezifisch für die Amplifikation einer Nucleinsequenz von Campylobacter coli, dadurch gekennzeichnet, daß sie Oligonucleotidpaare mit 18 bis 30 Nucleotiden, vorzugsweise 18 bis 22 Nucleotiden, ausgewählt aus den Sequenzen nach Anspruch 1, umfassen.

7. Oligonucleotidisches Primerpaar, spezifisch für die Amplifikation einer Nucleinsequenz von Campylobacter coli gemäß Anspruch 6, dadurch gekennzeichnet, daß es gebildet wird aus Oligonucleotidpaaren der Sequenzen:

8. Nucleinsäurefragment, erhalten durch Amplifikation einer Nucleinsäure von C. coli mittels einer Gen-Amplifikationtechnik, insbesondere mit der PCR-Technik mittels eines oligonucleotidischen Primerpaares nach Anspruch 6 oder 7.

9. Verfahren zur Bestimmung der Anwesenheit von Campylobacter coli in einer biologischen Probe, gekennzeichnet durch folgende Stufen:
a) Kontaktieren der biologischen Probe mit einem Primerpaar gemäß Anspruch 6 oder 7, wobei die Nucleinsäure von Campylobacter coli, die in der biologischen Probe enthalten ist, gegebenenfalls vorher für eine Hybridisierung zugänglich gemacht wurde, und unter Bedingungen, die eine Hybridisierung der Primer zu der Nucleinsäure, die Campylobacter coli angehört, ermöglichen;
b) Amplifikation der zu Campylobacter coli gehörenden Nucleinsäure;
c) Darstellung der Amplifikation der Nucleinsäurefragmente von Campylobacter coli, die dem durch die Primer umfaßten Fragment entsprechen;
d) gegebenenfalls Feststellung der Sequenz des amplifizierten Fragments, beispielsweise durch Hybridisierung mit spezifischer Sonde, durch Sequenzierung oder durch Analyse der Restriktionsstelle.

10. Kit zur Feststellung der Anwesenheit von Campylobacter coli in einer biologischen Probe, dadurch gekennzeichnet, daß es folgende Elemente umfaßt:
- ein Primerpaar nach Anspruch 6 oder 7;
- die zur Erwirkung einer Amplifikation einer Nucleinsäure von Campylobacter coli notwendigen Reagenzien;
- gegebenenfalls einen Bestandteil der die Feststellung der Sequenz des amplifizierten Fragments ermöglicht, insbesondere eine Nucleinsonde nach Anspruch 4 oder 5.

## Claims

1. Nucleic acid sequence which hybridises specifically with a genomic nucleic acid of the families belonging to the species *Campylobacter coli* and which does not hybridise under normal conditions with a nucleic acid of campylobacteria not belonging to this species, nor to a genomic nucleic acid of bacteria close to the *Campylobacter* genus, characterised in that it contains the nucleotide sequence SEQ ID N° 1, the sequence which is complementary to the latter, or a sequence which differs by mutation, insertion, deletion or substitution of one or more bases.

2. Cloning vector characterised in that it contains a nucleotide sequence according to claim 1.

3. Plasmid deposited in the National Collection of Microorganism Cultures on 14 November 1994 under the n°I-1491.

4. Specific nucleic probe of *Campylobacter coli* nucleic acids, characterised in that it comprises at least 20 consecutive nucleotides selected from among the nucleotide sequences according to claim 1.

5. Nucleic probe according to claim 4, characterised in that it is immobilised on a support and used as a capture probe.

6. Pairs of specific oligonucleotide primers for amplifying a *Campylobacter coli* nucleic acid sequence, characterised in that they comprise pairs of oligonucleotides having 18 to 30 nucleotides, preferably 18 to 22 nucleotides, selected from among the sequences according to claim 1.

7. Pair of specific oligonucleotide primers for amplifying a *Campylobacter coli* nucleic acid sequence according to claim 6, characterised in that the pairs of oligonucleotide sequences are constituted by:

8. Nucleic acid fragment obtained by amplifying a C. *coli* nucleic acid by a genetic amplifying technique, in particular by the PCR technique, by means of a pair of oligonucleotide primers according to claim 6 or 7.

9. Method of detecting the presence of *Campylobacter coli* in a biological sample, characterised by the following steps:
a) putting the biological sample in contact with a pair of primers according to claim 6 or 7, the *Campylobacter coli* nucleic acid contained in the biological sample having been, where appropriate, previously rendered accessible to hybridisation, and in conditions allowing hybridisation of the primers to the nucleic acid belonging to *Campylobacter coli ;*
b) amplifying the nucleic acid belonging to *Campylobacter coli ;*
c) detecting the amplification of *Campylobacter coli* nucleic acid fragments corresponding to the fragment framed by the primers;
d) possibly verifying the sequence of the amplified fragment, for example by hybridisation of the specific probe, by sequencing or by restriction site analysis.

10. Kit for detecting the presence of *Campylobacter coli* in a biological sample, characterised in that it comprises the following elements :
- a pair of primers according to claim 6 or 7
- the necessary reagents for effecting an amplification of a nucleic acid of *Campylobacter coli*
- possibly, a component permitting verification of the sequence of the amplified fragment, more particularly a nucleic probe according to claim 4 or 5.


### LISTE DE SEQUENCES

### (1) INFORMATION GENERALE:

(i) DEPOSANT:
   (A) NOM: INSTITUT PASTEUR
   (B) RUE: 28 RUE DU DOCTEUR ROUX
   (C) VILLE: PARIS
   (E) PAYS: FRANCE
   (F) CODE POSTAL: 75724
   (G) TELEPHONE: 45688093
   (H) TELECOPIE: 40613017
(ii) TITRE DE L' INVENTION: SEQENCES NUCLEOTIDIQUES HYBRIDANT SPECIFIQUEMENT AVEC UNE SEQUENCE NUCLEIQUE GENOMIQUE DE CAMPYLOBACTER COLI
(iii) NOMBRE DE SEQUENCES: 3
(iv) FORME LISIBLE PAR ORDINATEUR:
   (A) TYPE DE SUPPORT: Floppy disk
   (B) ORDINATEUR: IBM PC compatible
   (C) SYSTEME D' EXPLOITATION: PC-DOS/MS-DOS
   (D) LOGICIEL: PatentIn Release £1.0. Version £1.25 (OEB)
(v) DONNEES DE LA DEMANDE ACTUELLE: NUMERO DE DEPOT: WO PCT/FR95/01491

### (2) INFORMATION POUR LA SEQ ID NO: 1:

(i) CARACTERISTIQUES DE LA SEQUENCE:
   (A) LONGUEUR: 604 paires de bases
   (B) TYPE: acide nucléique
   (C) NOMBRE DE BRINS: double
   (D) CONFIGURATION: linéaire
(ii) TYPE DE MOLECULE: ADN (génomique)
(vi) ORIGINE:
   (A) ORGANISME: CS1
   (C) INDIVIDUEL ISOLE: CAMPYLOBACTER COLI
   (G) TYPE DE CELLULE: BACTERIE
(xi) DESCRIPTION DE LA SEQUENCE: SEQ ID NO: 1:
   TTAAAAAGAC TTATTTCCAT ACTAAATCAA CCCAAATAAA AATAAAAAAT ATTATACTTA    60
   AATATCCATT TAAAGTAAAA AACGCTTTAT CAATGTGAGC AAAATTTTTA TGTACAATTT   120
   TATGTTCAAA AGCCAAAATA ATACCGCTGA TAATCATTCC AAATAATGCA ATATTTCCAA   180
   GCGCTACTCC CCAAACCTGC CAAACAAAAA GTAGCCAAAA TAATACTGCT AATACATGGC   240
   AAAAGCCGAG ATAAATAAAG TCGCCTTAGA GCCAAATTTA GCAGGGATAG AATGAAGTCC   300
   TACTTTTTTA TCATACTCCA TATCTTGTAA AGAGTAAAGC AAATCAAATC CAGCCGTCCA   360
   AAAAGTTACC CCTAAGCATA AAATCACACT ATAAATGTGA ATTTCACCCA TGACTATCAC   420
   ACTGCCTGCA ATAGGTGCAA GTCCTAAACA AAATCCTAAA ACTAAATGCG CTAAAGACTA   480
   AAGCGTTTAA AAGCAGAATA AATTGCCAAT ACAAAAAGCA CAGGAAAAGA AAGATAAAAA   540
   GCCAAAGTAT TGATAAAATA AGAACACAAT ATAAAAATAA TCGCATTAGG ATAATAAAAT   600
   CCAA                                                                604

### (2) INFORMATION POUR LA SEQ ID No: 2:

(i) CARACTERISTIQUES DE LA SEQUENCE:
   (A) LONGUEUR: 22 paires de bases
   (B) TYPE: acide nucléique
   (C) NOMBRE DE BRINS: simple
   (D) CONFIGURATION: linéaire
(ii) TYPE DE MOLECULE: ADN (génomique)
(xi) DESCRIPTION DE LA SEQUENCE: SEQ ID NO: 2:
   ATATTTCCAA GCGCTACTCC CC 22

### (2) INFORMATION POUR LA SEQ ID NO: 3:

(i) CARACTERISTIQUES DE LA SEQUENCE:
   (A) LONGUEUR: 22 paires de bases
   (B) TYPE: acide nucléique
   (C) NOMBRE DE BRINS: simple
   (D) CONFIGURATION: linéaire
(ii) TYPE DE MOLECULE: ADN (génomique)
(xi) DESCRIPTION DE LA SEQUENCE: SEQ ID NO: 3:
   CAGGCAGTGT GATAGTCATG GG 22